# EUROPEAN PATENT SPECIFICATION

(11) **EP 2 122 331 B1**
(45) Date of publication and mention of the grant of the patent: **19.07.2017**
(21) Application number: 08706262.6
(22) Date of filing: 23.01.2008
(51) Int. Cl.: G01N 21/64, A61B 5/00, A61B 6/00, G01N 21/27, G01N 21/31, G01N 21/35, A61B 1/00, A61B 1/04, A61B 1/05

(54) **SYSTEM FOR MULTI- WAVELENGTH FLUORESCENCE AND REFLECTANCE IMAGING**
SYSTEM FÜR FLUORESZENZ- UND REFLEKTANZ-BILDGEBUNG BEI MEHREREN WELLENLÄNGEN
SYSTÈME D'IMAGERIE DE FLUORESCENCE ET DE RÉFLECTANCE À LONGUEURS D'ONDE MULTIPLES

(30) Priority: 23.01.2007 US 626308
(43) Date of publication of application: 25.11.2009
(73) Proprietor: Novadaq Technologies Inc., Mississauga, ON L4W 4T9 (CA)
(72) Inventor: CLINE, W., Richard, Vancouver, British Columbia V6R 1N2 (CA); FENGLER, J.P., John, North Vancouver, British Columbia V7G 2L8 (CA)
(74) Representative: V.O.
(86) International application number: PCT/CA2008/000115
(87) International publication number: WO 2008/089545

(56) References cited:
- WO-A2-02/50518
- US-A- 5 833 617
- US-A- 6 161 035
- US-A1- 2004 044 275
- US-A1- 2005 273 011
- US-B1- 6 603 552
- US-B1- 6 826 424
- US-B2- 6 821 245

## Description

### FIELD OF THE INVENTION

The present invention relates generally to the fields of diagnostic imaging. More particularly, it concerns methods and apparatus for generating multispectral images using fluorescence and reflectance imaging techniques.

### BACKGROUND OF THE INVENTION

Over the past 20 years, techniques of fluorescence imaging have been developed that utilize differences in the fluorescence response of normal tissue and tissue suspicious for early disease, such as cancer, as a tool in the detection and localization of such disease. The fluorescing compounds or fluorophores that are excited during fluorescence endoscopy may be exogenously applied photo active drugs that accumulate preferentially in suspicious tissues, or they may be endogenous fluorophores that are present in all tissue. In the latter case, the fluorescence from the tissue is typically referred to as autofluorescence or native fluorescence. Tissue autofluorescence is commonly due to fluorophores with absorption bands in the UV and blue portion of the visible spectrum and emission bands in the green to red portions of the visible spectrum. In tissue suspicious for early cancer, the cyan to green portion of the autofluorescence spectrum is usually significantly suppressed. Fluorescence imaging that is based on tissue autofluorescence utilizes this spectral difference to distinguish normal from suspicious tissue.

Representative fluorescence imaging systems that image drug induced fluorescence or tissue autofluorescence are disclosed in U.S. Patent Nos. 5,507,287, issued to Palcic et al.; 5,590,660, issued to MacAulay et al.; 5,827,190, issued to Palcic et al., U.S. Patent Application Serial No. 09/905,642, (US2002035330 A1) and U.S. Patent Application Serial No. 10/050,601, (US2003135092 A1). Each of these is assigned to Xillix Technologies Corp. of Richmond, British Columbia, Canada, the assignee of the present application.

While the systems disclosed in the above referenced patents are significant advances, improvements can be made. In particular, it is desirable to improve the specificity of fluorescence imaging, and to reduce the size, weight, and complexity of cameras, such that they can be miniaturized and built into the insertion portion of an endoscope.

US-A-5,833,617 discloses a fluorescence detecting apparatus. In use, a region of interest in a living body is exposed to excitation light, which causes it to produce fluorescence. The fluorescence is separated with respect to desired wavelength ranges and detected. A first detector detects an entire fluorescence component or a fluorescence sum component, which is the sum of a fluorescence component of a comparatively short wavelength region and a fluorescence component of a comparatively long wavelength region. A second detector detects a fluorescence component of a comparatively long wavelength region or a fluorescence difference component, which is the difference between a fluorescence component of a comparatively short wavelength region and a fluorescence component of a comparatively long wavelength region. The output from the first detector and the output from the second detector are divided by each other. The fluorescence intensity depending upon the distance with respect to the region of interest is thus corrected such that no error may occur in making an operation.

### SUMMARY OF THE INVENTION

A fluorescence imaging system in accordance with the present invention is as set out in claim 1.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 is a block diagram of a fluorescence imaging system according to one embodiment of the present invention;
FIGURE 2 is a block diagram of a multi mode light source in accordance with several embodiments of the present invention;
FIGURE 3 illustrates a camera that can acquire color and/or fluorescence/reflectance images. This camera is outside the scope of the invention.

Document US6821245 B2 discloses a fluorescence endoscopy video system includes a multimode light source that produces light for color and fluorescence imaging modes. Light from the light source is transmitted through an endoscope to the tissue under observation. The system also includes a camera for color and fluorescence imaging. Images obtained through the endoscope are optically divided and projected onto a single color image sensors, and optionally a low-light image sensor, by a fixed beam splitter in the camera. A first spectral filter limits the light transmitted to the one or more image sensors to either green or red autofluorescence light only and blocks the light in the excitation and reference wavebands transmitted by the light source. A second spectral filter is inserted into the optical path of the color image sensor and transmits only the reflected reference waveband light or autofluorescence light not transmitted by the first spectral filter.

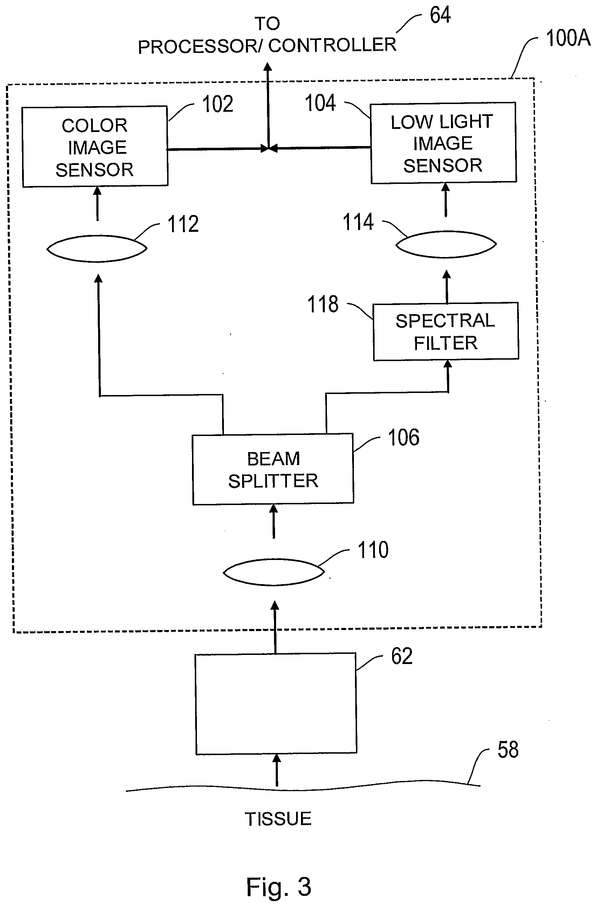

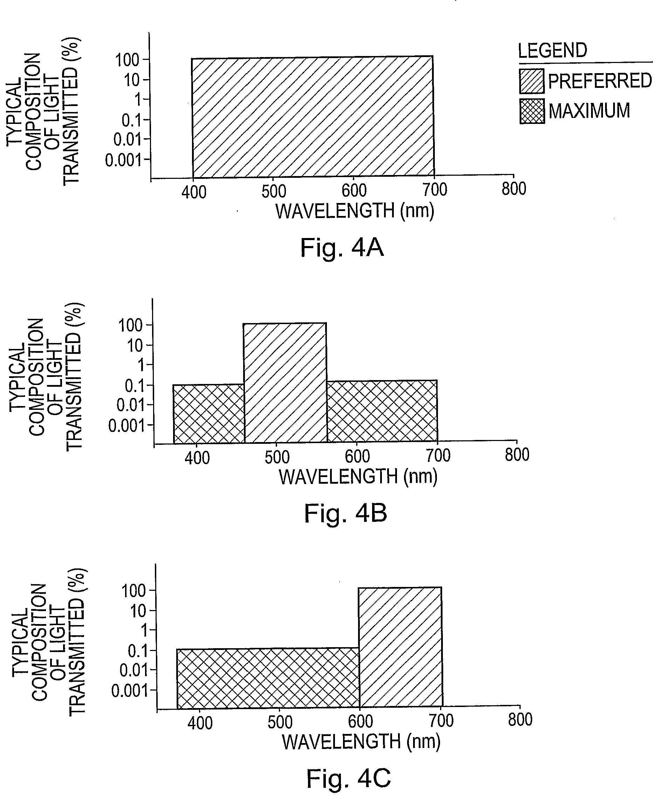

FIGURES 4A-4I are graphs illustrating suitable transmission characteristics of filters utilized for color imaging and fluorescence/reflectance imaging with the camera arrangement shown in FIGURE 3;

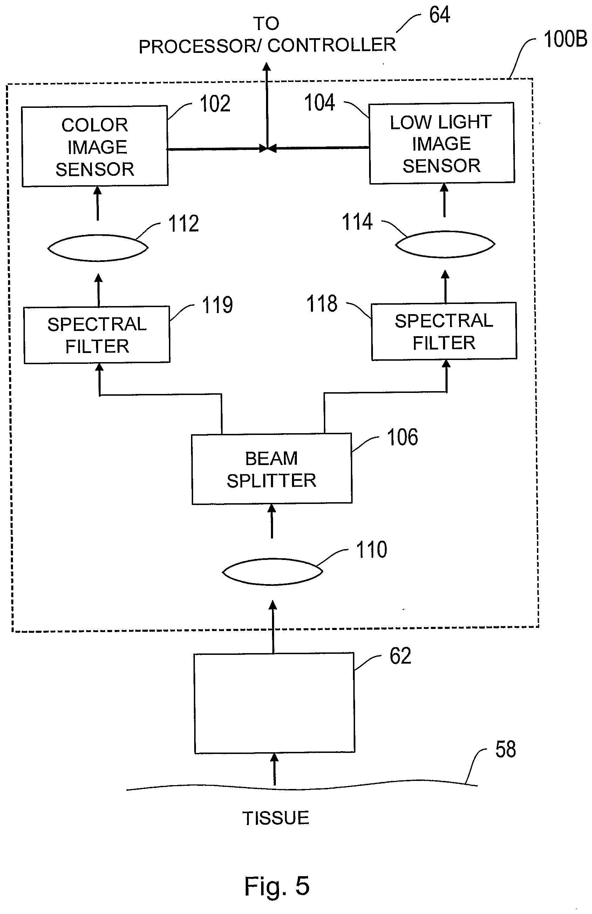

FIGURE 5 illustrates a camera like that of FIGURE 3 with an additional filter, this camera arrangement being outside the scope of the invention;

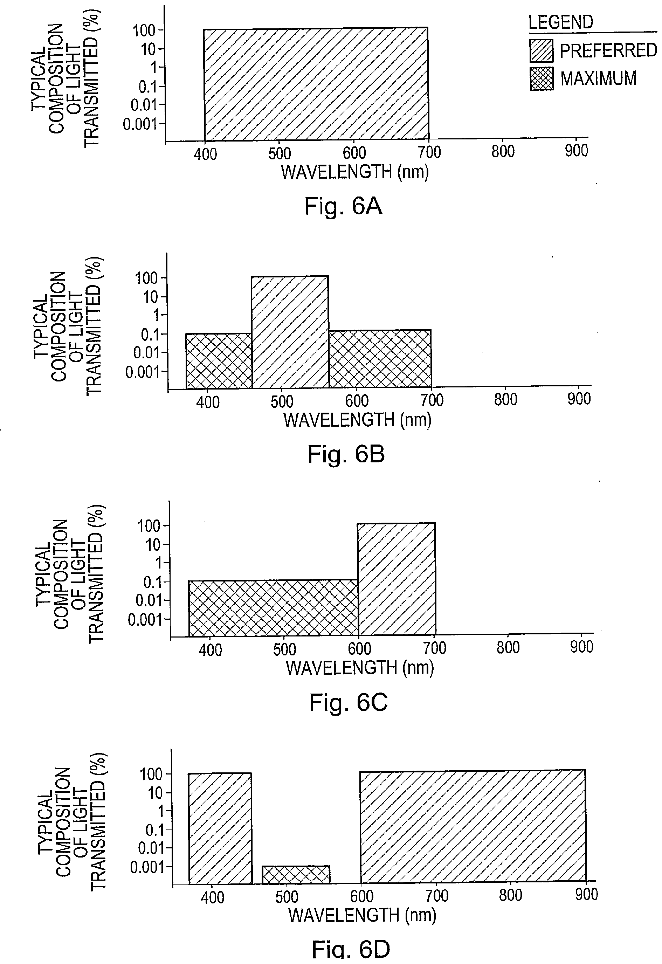

FIGURES 6A-6J are graphs illustrating suitable transmission characteristics of filters utilized for color imaging and fluorescence/reflectance imaging with the camera arrangement shown in FIGURE 5;

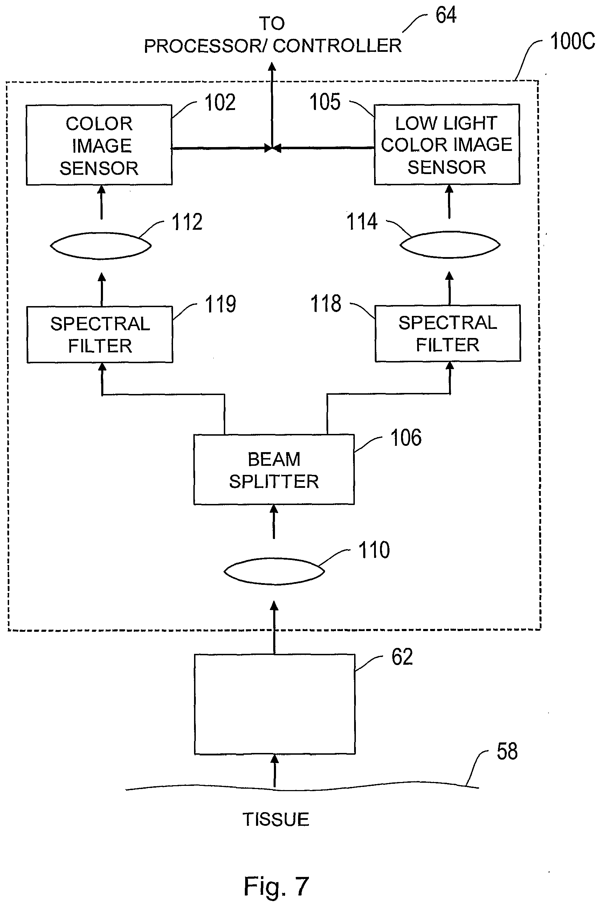

FIGURE 7 illustrates a camera like that of FIGURE 3 but with a low light color image sensor replacing the low light image sensor, according to one embodiment of the present invention; and

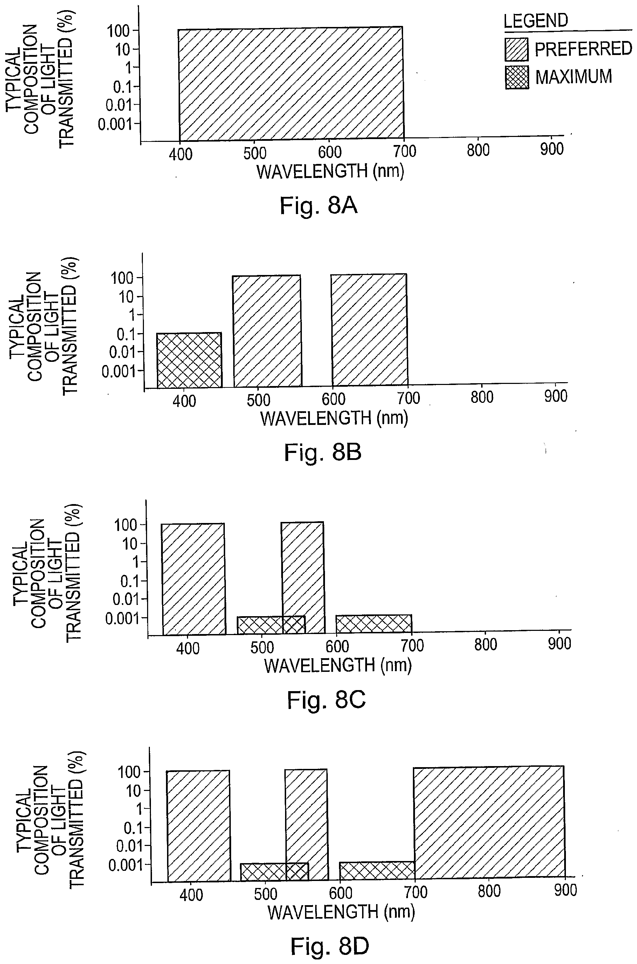

FIGURES 8A-8F are graphs illustrating presently preferred transmission characteristics of filters utilized for color imaging and fluorescence/reflectance imaging with the camera embodiment shown in FIGURE 7.

### DETAILED DESCRIPTION OF THE PREFERRED EMBODIMENT

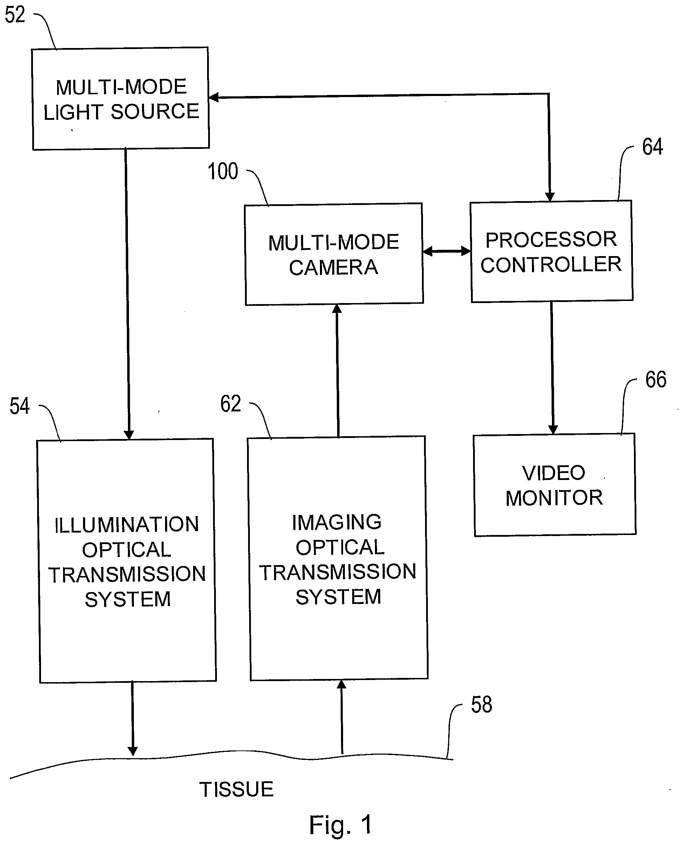

FIGURE 1 is a block diagram of a fluorescence and color imaging system 50 in accordance with one embodiment of the present invention. The system includes a multi mode light source 52 that generates light for obtaining color and fluorescence images. The use of the light source for obtaining different kinds of images will be described in further detail below. Light from light source 52 is supplied to an illumination optical transmission system 54, which then illuminates a tissue sample 58 that is to be imaged.

As shown in FIGURE 1, the system also includes an imaging optical transmission system 62 which transmits light from the tissue to a multi mode camera 100, that captures the light from the tissue. The camera can be utilized for fluorescence/reflectance imaging in additional to conventional color imaging. Fluorescence/reflectance imaging will be described in detail below.

A processor/controller 64 controls the multi-mode camera 100 and the light source 52, and produces video signals that are displayed on a video monitor 66.

The illumination optical transmission system 54 can consist of endoscope components, such as an endoscope illumination guide assembly. Alternatively, it can consist of the illumination optical system of a long working distance microscope, such as a colposcope. Similarly, the imaging optical transmission system 64 can consist of endoscope components, such as an endoscope image capturing optical assembly when camera 100 is located in the insertion portion of an endoscope. Alternatively, the imaging optical transmission system 64 can consist of the imaging optical system of a long working distance microscope, such as a colposcope.

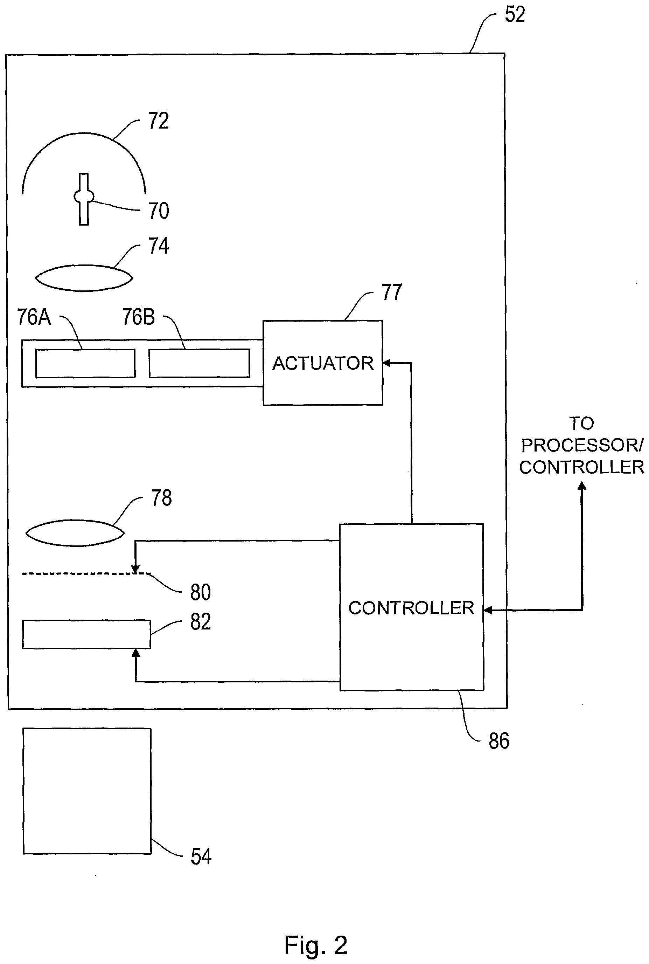

FIGURE 2 shows the components of the light source 52 in greater detail. The light source 52 includes an arc lamp 70 that is surrounded by a reflector 72. In the preferred embodiment of the invention, the arc lamp 70 is a high pressure mercury arc lamp (such as the Osram VIP R 150/P24). Alternatively, other arc lamps, solid state devices (such as light emitting diodes or diode lasers), or broadband light sources may be used, but a high pressure mercury lamp is currently preferred for its combination of high blue light output, reasonably flat white light spectrum, and small arc size.

The light from the arc lamp 70 is coupled to illumination optical transmission system 54 through appropriate optical components 74, 76, and 78 for light collection, spectral filtering and focusing respectively. The light from the arc lamp is spectrally filtered by one of a number of optical filters 76A, 76B, ... that operate to pass or reject desired wavelengths of light in accordance with the operating mode of the system. As used herein, "wavelength" is to be interpreted broadly to include not only a single wavelength, but a range of wavelengths as well. A controller 86 operates an actuator 77 that moves the filters 76A, 76B, ... into and out of the light path.

The optical filter characteristics of filters 76A, 76B ... are tailored for each imaging mode. For example, optical filter 76A, used for color imaging, reduces any spectral peaks and modifies the color temperature of the arc lamp 70 so that the output spectrum simulates sunlight. Optical filter 76B transmits both fluorescence excitation light and reflectance light for use with the fluorescence/reflectance imaging mode. The transmission characteristics of the light source filters are described in more detail below in the context of the various camera embodiments.

Because fluorescence imaging is generally used in conjunction with color imaging, each of the various multi-mode cameras 100 described below may be used both for color and fluorescence/reflectance imaging.

In a first arrangement, outside the scope of the invention, shown in FIGURE 3, a camera 100A receives light from the tissue 58, by means of the imaging optical transmission system 62 that transmits the light from the tissue to the camera, as shown in FIGURE 1. The light is directed toward a color image sensor 102 and a low light image sensor 104 by a fixed optical beamsplitter 106 that splits the incoming light into two beams. The beamsplitter may be a standard commercially available single plate, single cube, or single pellicle design. It should be noted that, if the optical path between the tissue 58 and the image sensors contains an uneven number of reflections (e.g., such as from a single component beamsplitter), the image projected onto the sensor will be left to right inverted. The orientation of such images will need to be corrected through image processing.

In FIGURE 3, light collimating optics 110 are positioned in front of the beamsplitter 106, and imaging optics 112 and 114 are positioned immediately preceding the color image sensor 102 and the low light image sensor 104, respectively. These optical elements are optional, with the need for the collimating optics 110 depending on the optical characteristics of the imaging optical transmission system 62, and the need for imaging optics 112 and 114 depending on whether or not all beam paths are same length. A spectral filter 118 is located in the optical path between the beamsplitter 106 and the low light image sensor 104. Alternatively, the spectral filter 118 may be incorporated as an element of the beamsplitter 106.

The low light image sensor 104 preferably comprises a (monochrome) charge coupled device (CCD) with charge carrier multiplication (of the same type as the Texas Instruments TC253 or the Marconi Technologies CCD65), electron beam charge coupled device (EBCCD), intensified charge coupled device (ICCD), charge injection device (CID), charge modulation device (CMD), complementary metal oxide semiconductor image sensor (CMOS) or charge coupled device (CCD) type sensor. The color image sensor 102 is preferably a CCD or a CMOS image sensor incorporating integrated mosaic filters.

Based on operator input, the processor/controller 64 also provides control functions for the fluorescence imaging system. These control functions include providing control signals that control the camera gain in all imaging modes, coordinating the imaging modes of the camera and light source, and providing a light level control signal for the light source.

The nature of the fluorescence/reflectance imaging, will now be explained. It is known from in vivo spectroscopy that the intensity of the autofluorescence at certain wavelengths changes as tissues become increasingly abnormal (i.e., as they progress from normal to frank cancer). When visualizing images formed from such a band of wavelengths of autofluorescence, however, it is not easy to distinguish between those changes in the signal strength that are due to pathology and those that are due to imaging geometry and shadows. A reflected light image acquired in a band of wavelengths in which the image signal is not significantly affected by tissue pathology consisting of light that has undergone scattering within the tissue (known as diffuse reflectance) may be used as a reference signal for fluorescence/reflectance imaging with which the signal strength of the first fluorescence image can be "normalized". Such normalization is described in U.S. Patent No. 5,590,660, issued to MacAulay et al. discussed above.

One technique described in the '660 patent for performing the normalization is to assign each of the two image signals a different display color, e.g., by supplying the image signals to different color inputs of a color video monitor. When displayed in this manner on a color video monitor, the two images are effectively combined by the user's visual system to form a single image, the combined color of which represents the relative strengths of the signals from the two images. The mixture of colors with which normal tissue and tissue suspicious for early cancer are displayed depends on the gain applied to each of the two separate image signals. Since light originating from fluorescence within tissue and diffuse reflectance light which has undergone scattering within the tissue are both emitted from the tissue with a similar spatial distribution of intensities, the color of a combined image is independent of the absolute strength of the separate image signals, and will not change as a result of changes in the distance or angle to the tissue sample 58, or changes in other imaging geometry factors. If, however, there is a change in the shape of the autofluorescence spectrum of the observed tissue that gives rise to a change in the relative strength of the two image signals, such a change will be represented as a change in the color of the displayed image.

The present invention goes beyond fluorescence/reflectance imaging as described in the '660 patent to take advantage of additional information about the disease state of tissue contained in reflected light by making use of more than one reflectance image for fluorescence imaging. As shown in FIGURE 3, during fluorescence imaging, the low light image sensor 104 transduces light that has been filtered by spectral filter 118. This sensor/filter combination is utilized to capture a fluorescence image. The color image sensor 102 transduces light filtered by its integrated mosaic filters and is utilized to capture images from up to three different bands of wavelengths of reflected light. These bands of wavelengths of reflected light can be bands in which the image signal is not significantly affected by tissue pathology as described in the '660 patent, or they can bands of wavelengths containing information about the disease state of the tissue.

In vivo spectroscopy has been used to determine which differences in tissue autofluorescence and reflectance spectra have a pathological basis. The properties of these spectra determine the particular wavelength bands of autofluorescence and reflected light that can be utilized to provide improved discrimination of disease in the fluorescence/reflectance imaging mode. Since the properties of the spectra depend on the tissue type, the wavelengths of the important autofluorescence and reflectance bands may depend on the type of tissue being imaged. The specifications of the optical filters described below are a consequence of these spectral characteristics, and are chosen to be optimal for the tissues to be imaged.

The intensity of diffuse reflected light at a given wavelength varies with pathology for a number of reasons, including differences in light absorption arising from changes in tissue oxygenation and differences in Mie scattering arising from changes in the size of cell nuclei. It is well known that cancerous tissue is hypoxic and contains more hemoglobin than oxy-hemoglobin compared to normal tissue. The intensity of light reflected from tissue is affected by hemoglobin and oxy-hemoglobin which strongly absorb visible light. The relative abundance of hemoglobin and oxy-hemoglobin can be determined from reflected light utilizing wavelengths corresponding to maxima in the respective absorption spectra. In the visible region, these absorption maxima occur at approximately 435 nm and 555 nm for hemoglobin and at 415 nm, 542 nm, and 576 nm for oxy-hemoglobin. In the red/NIR region, absorption is stronger for hemoglobin at wavelengths shorter than 797 nm and stronger for oxy-hemoglobin at wavelengths longer than 797 nm. By utilizing bands of wavelengths near these absorption maxima, diffuse reflectance images can be captured with the camera shown in FIGURE 3 that provide information about the relative abundance of hemoglobin and oxy-hemoglobin the tissue.

There are several possible configurations of fluorescence/reflectance imaging that can be utilized with camera 100A shown in FIGURE 3, including cyan/green fluorescence with either (a) red/NIR and violet/blue reflectance, (b) violet/blue and green/yellow reflectance, or (c) violet/blue, green/yellow, and red/NIR reflectance or cyan/green fluorescence with green/yellow reflectance and red/NIR reflectance. Alternatively the camera 100A can use red fluorescence with either (i) NIR and violet/blue reflectance, (ii) green/yellow/orange or violet/blue reflectance, or (iii) NIR, green/yellow, and violet/blue reflectance or (iv) red fluorescence with green/yellow reflectance and NIR reflectance The particular configuration utilized depends on the target clinical organ and application.

In the present arrangement, the band of wavelengths utilized to detect fluorescence is defined by filter 118 shown in FIGURE 3, and the bands of wavelengths utilized to detect reflectance are defined by the combination of the mosaic filters integrated in color image sensor 102 shown in FIGURE 3 and light source filter 76B shown in FIGURE 2. The mosaic filters in color image sensor 102 typically have very broad passbands, therefore, if narrow bands of wavelengths are to be utilized, they are defined by light source filter 76B.

An additional requirement on light source filter 76B arises from the use of one sensor to capture multiple reflectance images. In order to effectively capture multiple reflectance images with the same color image sensor 102 shown in FIGURE 3, the intensity of the reflected light received at the sensor should be approximately the same in each band of wavelengths to be detected. In the present arrangement, shown in FIGURE 3, the relative intensity of the reflected light at the color image sensor is controlled by the design of the light source filter 76B shown in FIGURE 2.

FIGURES 4A-4I illustrate suitable filter characteristics for use in a fluorescence and color imaging system having a camera of the type shown in FIGURE 3 and light source as shown in FIGURE 2, that operates in a fluorescence/reflectance imaging mode, or a color imaging mode.

FIGURE 4A illustrates the composition of light transmitted by the light source filter, such as filter 76A, which is used to produce light for color imaging. This filter produces white light for use in color imaging by attenuating undesired peaks in the lamp spectrum and by correcting the color temperature of the light from the lamp.

FIGURE 4B illustrates the composition of the light transmitted by camera filter 118 for the detection of fluorescence at cyan and green wavelengths. Used in this configuration, the filter blocks violet/blue excitation light in the range 370 - 455 nm while transmitting cyan/green light in the wavelength range of 470 - 560 nm or any desired subset of wavelengths in this range at the maximum possible transmission. When used in a fluorescence and color imaging system for fluorescence/reflectance imaging, in combination with light source filter 76B described below, the filter characteristics are such that any light outside of the wavelength range of 470 nm - 560 nm (or any desired subset of wavelengths in this range) contributes no more than 0.1% to the light transmitted by the filter.

FIGURE 4C illustrates the composition of the light transmitted by camera filter 118 for the detection of fluorescence at red wavelengths. Used in this configuration, the filter blocks violet/blue excitation light in the range 370 - 455 nm while transmitting red light in the wavelength range of 600 - 700 nm or any desired subset of wavelengths in this range at the maximum possible transmission. When used in a fluorescence and color imaging system for fluorescence/reflectance imaging, in combination with light source filter 76B described below, the filter characteristics are such that any light outside of the wavelength range of 600 nm - 700 nm (or any desired subset of wavelengths in this range) contributes no more than 0.1% to the light transmitted by the filter.

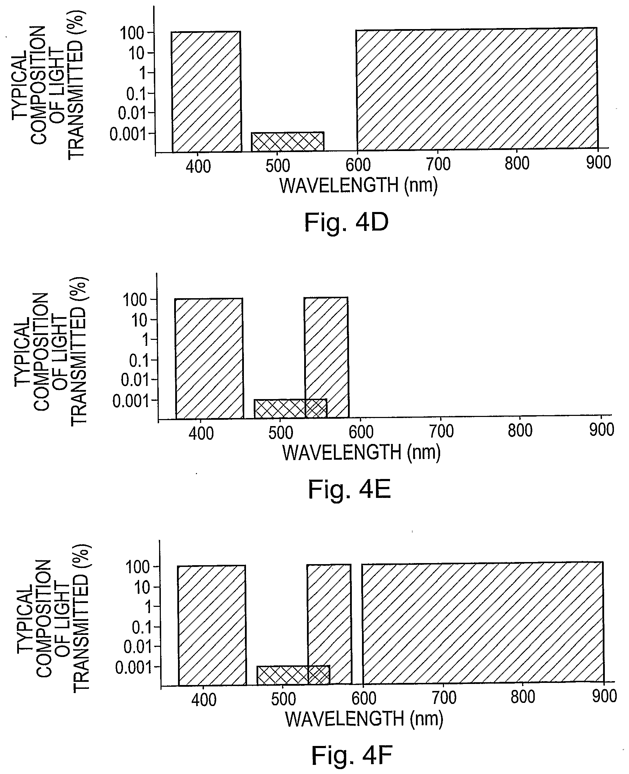

FIGURE 4D illustrates the composition of the light transmitted by light source filter 76B which is used to produce light for fluorescence excitation and reflectance imaging at violet/blue and red/NIR wavelengths and fluorescence imaging in the cyan/green. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the red/NIR wavelength range of 600 - 900 nm, or any subset of wavelengths in this range (in particular 600 - 797 nm for a hemoglobin reflectance image and 797 - 900 nm for an oxy-hemoglobin reflectance image). Of the light transmitted by the filter, less than 0.001% is in the cyan/green fluorescence imaging wavelength range of 470 - 560 nm (or whatever desired subset of this range is specified as the transmission range of the primary fluorescence wavelength band). The light transmitted in the red/NIR wavelength range is adjusted, as part of the system design, to be an appropriate fraction of the light transmitted in the violet/blue wavelength band such that the light projected onto the color image sensor in each of these bands has comparable intensity.

FIGURE 4E illustrates the composition of light transmitted by the light source filter, such as filter 76B, which is used to produce light for fluorescence excitation and reflectance imaging at violet/blue and green/yellow wavelengths and fluorescence imaging in the cyan/green. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). Of the light transmitted by the filter, less than 0.001% is in the cyan/green fluorescence imaging wavelength range of 470 - 560 nm (or whatever desired subset of this range is specified as the transmission range of the primary fluorescence wavelength band). The light transmitted in the green/yellow wavelength range is adjusted, as part of the system design, to be an appropriate fraction of the light transmitted in the violet/blue wavelength band such that the light projected onto the color image sensor in each of these bands has comparable intensity.

FIGURE 4F illustrates the composition of light transmitted by the light source filter, such as filter 76B, which is used to produce light for fluorescence excitation and reflectance imaging at violet/blue, green/yellow, and red/NIR wavelengths and fluorescence imaging in the cyan/green. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). In addition, it transmits light in the red/NIR wavelength range of 600 - 900 nm, or any desired subset of wavelengths in this range (in particular 700 - 797 nm for a hemoglobin reflectance image and 797 - 900 nm for an oxy-hemoglobin reflectance image). Of the light transmitted by the filter, less than 0.001% is in the cyan/green fluorescence imaging wavelength range of 470 - 560 nm (or whatever desired subset of this range is specified as the transmission range of the primary fluorescence wavelength band). The light transmitted in the red/NIR and green/yellow wavelength ranges is adjusted, as part of the system design, to be an appropriate fraction of the light transmitted in the violet/blue wavelength band such that the light projected onto the color image sensor in each of these bands has comparable intensity.

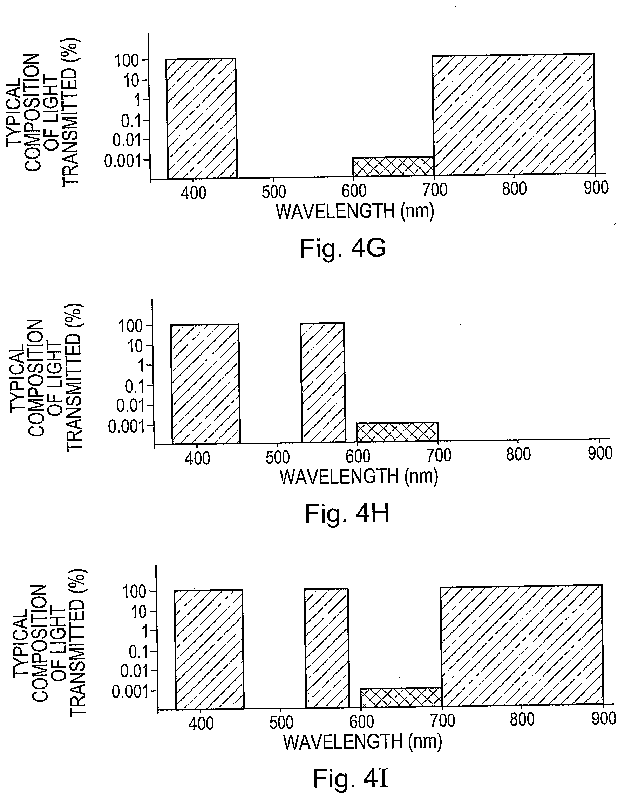

FIGURE 4G illustrates the composition of light transmitted by the light source filter, such as filter 76B, which is used to produce light for fluorescence excitation and reflectance imaging at NIR and violet/blue wavelengths and fluorescence imaging in the red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the NIR wavelength range of 700 - 900 nm, or any subset of wavelengths in this range (in particular 600 **700**- 797 nm for a hemoglobin reflectance image and 797 - 900 nm for an oxy-hemoglobin reflectance image). Of the light transmitted by the filter, less than 0.001% is in the red fluorescence imaging wavelength range of 600 - 700 nm (or whatever desired subset of this range is specified as the transmission range of the primary fluorescence wavelength band). The light transmitted in the NIR wavelength range is adjusted, as part of the system design, to be an appropriate fraction of the light transmitted in the violet/blue wavelength band such that the light projected onto the color image sensor in each of these bands has comparable intensity.

FIGURE 4H illustrates the composition of light transmitted by the light source filter, such as filter 76B, which is used to produce light for fluorescence excitation and reflectance imaging at green/yellow/orange and violet/blue wavelengths and fluorescence imaging in the red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). Of the light transmitted by the filter, less than 0.001% is in the red fluorescence imaging wavelength range of 600 - 700 nm (or whatever desired subset of this range is specified as the transmission range of the primary fluorescence wavelength band). The light transmitted in the green/yellow wavelength range is adjusted, as part of the system design, to be an appropriate fraction of the light transmitted in the violet/blue wavelength band such that the light projected onto the color image sensor in each of these bands has comparable intensity.

FIGURE 4I illustrates the composition of light transmitted by the light source filter, such as filter 76B, which is used to produce light for fluorescence excitation and reflectance imaging at NIR, green/yellow, and violet blue wavelengths and fluorescence imaging in the red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). In addition, it transmits light in the NIR wavelength range of 700 - 900 nm, or any desired subset of wavelengths in this range (in particular 700 - 797 nm for a hemoglobin reflectance image and 797 - 900 nm for an oxy-hemoglobin reflectance image). Of the light transmitted by the filter, less than 0.001% is in the red fluorescence imaging wavelength range of 600 - 700 nm (or whatever desired subset of this range is specified as the transmission range of the primary fluorescence wavelength band). The light transmitted in the NIR and green/yellow wavelength ranges is adjusted, as part of the system design, to be an appropriate fraction of the light transmitted in the violet/blue wavelength band such that the light projected onto the color image sensor in each of these bands has comparable intensity.

The operation of a system based on camera 100A of FIGURE 3 will now be described. The camera 100A is capable of operation in the color and fluorescence/reflectance imaging modes. For a system based on camera 100A, the light source shown in FIGURE 2 provides steady state output in each imaging mode.

In the color imaging mode, the processor/controller 64 provides a control signal to the multimode light source 52 that it should be in white light mode. The light source selects and positions the appropriate optical filter 76A into the optical path between the arc lamp 70 and endoscope light guide 54. The filtered light from the light source 52 is projected into the illumination optical transmission system and transmitted to illuminate the tissue 58.

Light reflected by tissue 58 is collected and transmitted by the imaging optical transmission system to the camera where it is projected through beamsplitter 106 onto the color image sensor 102 and the low light image sensor 104. Signals from low light image sensor 104 are not utilized during color imaging and processor/controller 64 protects the sensitive low light image sensor 104 by decreasing the gain of the amplification stage of the sensor. Image signals from the color image sensor 102 are processed by processor/controller 64. Standard techniques are utilized to produce a color image from a single color sensor: the image signals from pixels having the same filter characteristics are interpolated by processor/controller 64 to produce an image signal, related to the pass band of each element of the mosaic filter (e.g. red, green, and blue), at every pixel location. If the light beam undergoes an odd multiple of reflections on the path to color image sensor 102, the image is also inverted. The resulting multiple images, which when combined produce a color image, are encoded by processor/controller 64 as video signals. The color image is displayed by connecting the video signals to the appropriate inputs of color video monitor 66.

Processor/controller 64 also maintains the overall image brightness at a set level by monitoring the brightness of the image signal at each pixel and adjusting the intensity of the light source output and camera amplifier gains according to a programmed algorithm.

When switching to the fluorescence/reflectance imaging mode, processor/controller 64 provides a control signal to the multi-mode light source 52 to indicate that it should be in fluorescence/reflectance mode. The light source 52 moves light source filter 76B into position in the light beam. Filter 76B transmits both excitation light and reflectance light and blocks the transmission of light at fluorescence detection wavelengths, as described above. The filtered light from the light source 52 is projected into the illumination optical transmission system and transmitted to illuminate the tissue 58. Processor/controller 64 increases the gain of the amplification stage of the low light image sensor 104.

The fluorescence emitted and light reflected by tissue 58 is collected and transmitted by the imaging optical transmission system to the camera where it is projected through beamsplitter 106 onto the color image sensor 102 and the low light image sensor 104. Spectral filter 118 limits the light transmitted to the low light image sensor 104 to either cyan/green or red autofluorescence light only and substantially blocks the light in the excitation wavelength band. The fluorescence is transduced by low light sensor 104. Reflected light is transduced by color image sensor 102. The reflectance images from color image sensor 102 are processed, as previously described for color imaging, by processor/controller 64 to produce separate images corresponding to each of the pass bands of the mosaic filter (e.g. red, green, and blue). These separate reflectance images are encoded, along with the fluorescence signal from low light image sensor 104, as video signals by processor/controller 64. A composite fluorescence/reflectance image is produced by overlaying the fluorescence image and two or more reflectance images displayed in different colors on color video monitor 66. Alternatively, processor/controller 64 can produce a composite fluorescence/reflectance image by taking the difference between, or calculating the ratio of, two images, preferably one which changes with disease and one which does not change with disease or one affected by hemoglobin and one affected by oxy-hemoglobin and overlaying the resulting image, along with the fluorescence image and reflectance images.

As in the case of color imaging, during fluorescence/reflectance imaging processor/controller 64 maintains the overall image brightness at a set level by monitoring the brightness of the image signal at each pixel and adjusting the intensity of the light source output and camera amplifier gains according to a programmed algorithm.

FIGURE 5 illustrates another arrangement of the camera 100. Camera 100B is the same as camera 100A described above except that spectral filter 119 has been added to the light path of color image sensor 102. The advantage of this configuration is that a wide band of wavelengths can be utilized for fluorescence excitation (e.g. 390 - 455 nm) to produce a stronger fluorescence signal, independent of the width of the violet/blue band of wavelengths utilized in the detection of reflected light. Fairly narrow bands should be used for reflected light, if the light to be detected is to show the affect of absorption by only hemoglobin or only oxy-hemoglobin. Camera 100A in the arrangement of Fig. 3 necessitates the use of the same wavelengths of light for both fluorescence excitation and for the detection of violet/blue reflected light, which limits the amount of fluorescence that can be excited. Camera 100B allows excitation of the maximum possible fluorescence while allowing the detection of narrow bands of reflected light.

As discussed above, the filters in the light source and camera should be optimized for the imaging mode of the camera, the type of tissue to be examined and/or the type of pre cancerous tissue to be detected, based on in vivo spectroscopy measurements. The preferred filter characteristics for use in the fluorescence imaging systems with a camera of the type shown in FIGURE 2 and light source as shown in FIGURE 2, operating in a fluorescence/reflectance imaging mode and color imaging mode are shown in FIGURES 6A-6J. Like the arrangement described with reference to FIGURES 3 and 4A-4I, there are multiple possible configurations of such a fluorescence imaging system, operating in the fluorescence/reflectance imaging mode including cyan/green fluorescence with combinations of red/NIR, green/yellow, and violet/blue reflectance, and red fluorescence with combinations of NIR, green/yellow and violet/blue reflectance. The particular configuration utilized depends on the target clinical organ and application. The filter characteristics will now be described for each of these configurations.

FIGURE 6A illustrates the composition of light transmitted by the light source filter, such as filter 76A, which is used to produce light for color imaging. This filter produces white light for use in color imaging by attenuating undesired peaks in the lamp spectrum and by correcting the color temperature of the light from the lamp.

FIGURE 6B illustrates the composition of the light transmitted by camera spectral filter 118 for the detection of fluorescence at cyan/green wavelengths. Used in this configuration, the filter blocks violet/blue excitation light in the range 370 - 455 nm while transmitting cyan/green light in the wavelength range of 470 - 560 nm or any desired subset of wavelengths in this range at the maximum possible transmission. When used in a fluorescence and color imaging system for fluorescence/reflectance imaging, in combination with light source filter 76B described below, the filter characteristics are such that any light outside of the wavelength range of 470 nm - 560 nm (or any desired subset of wavelengths in this range) contributes no more than 0.1% to the light transmitted by the filter.

FIGURE 6C illustrates the composition of the light transmitted by camera spectral filter 118 for the detection of fluorescence at red wavelengths. Used in this configuration, the filter blocks violet/blue excitation light in the range 370 - 455 nm while transmitting red light in the wavelength range of 600 - 700 nm or any desired subset of wavelengths in this range at the maximum possible transmission. When used in a fluorescence and color imaging system for fluorescence/reflectance imaging, in combination with light source filter 76B described below, the filter characteristics are such that any light outside of the wavelength range of 600 nm - 700 nm (or any desired subset of wavelengths in this range) contributes no more than 0.1% to the light transmitted by the filter.

FIGURE 6D illustrates the composition of the light transmitted by light source filter 76B, which is used to produce light for fluorescence excitation and reflectance imaging at violet/blue and red/NIR wavelengths and fluorescence imaging in the cyan/green. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the red/NIR wavelength range of 600 - 900 nm, or any subset of wavelengths in this range (in particular 600 - 797 nm for a hemoglobin reflectance image and 797 - 900 nm for an oxy-hemoglobin reflectance image). Of the light transmitted by the filter, less than 0.001% is in the cyan/green fluorescence imaging wavelength range of 470 - 560 nm (or whatever desired subset of this range is specified as the transmission range of the primary fluorescence wavelength band). The light transmitted in the red/NIR wavelength range is adjusted, as part of the system design, to be an appropriate fraction of the light transmitted in the violet/blue wavelength band such that the light projected onto the color image sensor in each of these bands has comparable intensity.

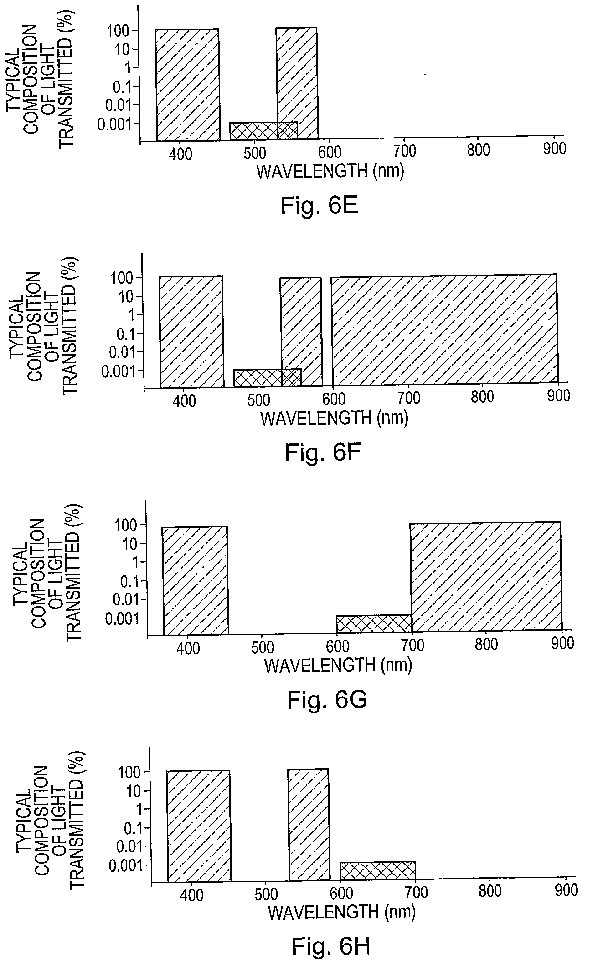

FIGURE 6E illustrates the composition of light transmitted by the light source filter 76B, which is used to produce light for fluorescence excitation and reflectance imaging at violet/blue and green/yellow wavelengths and fluorescence imaging in the cyan/green. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). Of the light transmitted by the filter 76B, less than 0.001% is in the cyan/green fluorescence imaging wavelength range of 470 - 560 nm (or whatever desired subset of this range is specified as the transmission range of the primary fluorescence wavelength band). The light transmitted in the green/yellow wavelength range is adjusted, as part of the system design, to be an appropriate fraction of the light transmitted in the violet/blue wavelength band such that the light projected onto the color image sensor, after passing through spectral filter 119, in each of these bands has comparable intensity.

FIGURE 6F illustrates the composition of light transmitted by the light source filter 76B, which is used to produce light for fluorescence excitation and reflectance imaging at violet/blue, green/yellow, and red/NIR wavelengths and fluorescence imaging in the cyan/green. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images except for light in the desired fluorescence spectral band). In addition, it transmits light in the red/NIR wavelength range of 600 - 900 nm, or any desired subset of wavelengths in this range (in particular 600 - 797 nm for a hemoglobin reflectance image and 797 - 900 nm for an oxy-hemoglobin reflectance image). Of the light transmitted by the filter, less than 0.001% is in the cyan/green fluorescence imaging wavelength range of 470 - 560 nm (or whatever desired subset of this range is specified as the transmission range of the primary fluorescence wavelength band). The light transmitted in the red/NIR and green/yellow wavelength ranges is adjusted, as part of the system design, to be an appropriate fraction of the light transmitted in the violet/blue wavelength band such that the light projected onto the color image sensor, after passing through spectral filter 119, in each of these bands has comparable intensity.

FIGURE 6G illustrates the composition of light transmitted by the light source filter 76B, which is used to produce light for fluorescence excitation and reflectance imaging at NIR and violet/blue wavelengths and fluorescence imaging in the red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the NIR wavelength range of 700 - 900 nm, or any subset of wavelengths in this range (in particular 700 - 797 nm for a hemoglobin reflectance image and 797 - 900 nm for an oxy-hemoglobin reflectance image). Of the light transmitted by the filter, less than 0.001% is in the red fluorescence imaging wavelength range of 600 - 700 nm (or whatever desired subset of this range is specified as the transmission range of the primary fluorescence wavelength band). The light transmitted in the NIR wavelength range is adjusted, as part of the system design, to be an appropriate fraction of the light transmitted in the violet/blue wavelength band such that the light projected onto the color image sensor in each of these bands has comparable intensity.

FIGURE 6H illustrates the composition of light transmitted by the light source filter 76B, which is used to produce light for fluorescence excitation and reflectance imaging at green/yellow, and violet blue wavelengths and fluorescence imaging in the red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). Of the light transmitted by the filter, less than 0.001% is in the red fluorescence imaging wavelength range of 600 - 700 nm (or whatever desired subset of this range is specified as the transmission range of the primary fluorescence wavelength band). The light transmitted in the green/yellow wavelength range is adjusted, as part of the system design, to be an appropriate fraction of the light transmitted in the violet/blue wavelength band such that the light projected onto the color image sensor in each of these bands has comparable intensity.

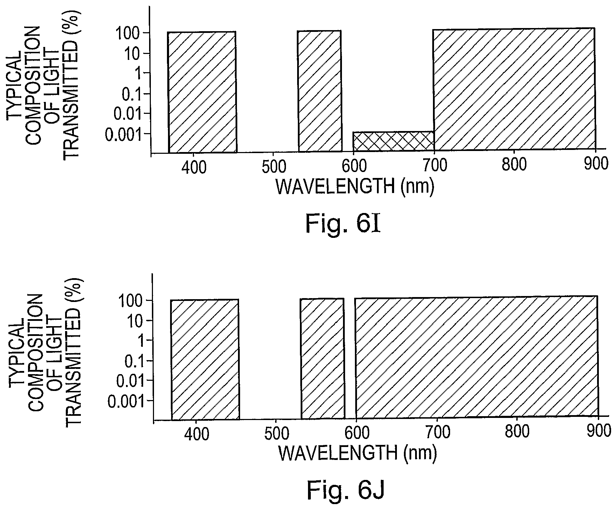

FIGURE 6I illustrates the composition of light transmitted by the light source filter 76B, which is used to produce light for fluorescence excitation and reflectance imaging at NIR, green/yellow, and violet blue wavelengths and fluorescence imaging in the red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). In addition, it transmits light in the NIR wavelength range of 700 - 800 nm, or any desired subset of wavelengths in this range. Of the light transmitted by the filter, less than 0.001 % is in the red fluorescence imaging wavelength range of 600 - 700 nm (or whatever desired subset of this range is specified as the transmission range of the primary fluorescence wavelength band). The light transmitted in the NIR and green/yellow wavelength ranges is adjusted, as part of the system design, to be an appropriate fraction of the light transmitted in the violet/blue wavelength band such that the light projected onto the color image sensor in each of these bands has comparable intensity.

FIGURE 6J illustrates the composition of the light transmitted by spectral filter 119 which is used to produce light for reflectance imaging at any combination of violet/blue, green/yellow and red/NIR wavelengths. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). In addition, it transmits light in the red/NIR wavelength range of 600 - 900 nm, or any desired subset of wavelengths in this range (in particular 600 - 797 nm for a hemoglobin reflectance image and 797 - 900 nm for an oxy-hemoglobin reflectance image). When used in a fluorescence and color imaging system for fluorescence/reflectance imaging, in combination with light source filter 76B described above, the filter characteristics are such that any light outside of the violet/blue, green/yellow, or red/NIR wavelength ranges specified above (or any desired subset of wavelengths in those ranges) contributes no more than 0.1% to the light transmitted by the filter. The light transmitted in the red/NIR, green/yellow, and violet/blue wavelength ranges is adjusted, as part of the system design, to be such that when a gray surface illuminated by white light filtered by light source filter 76A is imaged by color image sensor 102, the resulting color image may be white balanced.

It is possible that the band of wavelengths utilized to detect fluorescence is defined by filter 118 shown in FIGURE 5, and the bands of wavelengths utilized to detect reflectance are defined by the combination of the mosaic filters integrated in color image sensor 102 shown in FIGURE 5, light source filter 76B shown in FIGURE 2, and spectral filter 119. It is desired to have narrow pass bands for the detection of reflected light that is affected by the absorption of hemoglobin or oxy-hemoglobin alone, and at the same time use a broad band of wavelengths to maximize fluorescence excitation. This can be accomplished by controlling the width of the bands of wavelengths of reflected light using filter 119 (the mosaic filters in color image sensor 102 typically have very broad pass bands) and controlling the width of the band of wavelengths of fluorescence excitation light using light source filter 76B.

Two additional filter requirements arise from the use of one sensor to capture multiple reflectance images: 1) In order to be able to produce white balanced color images, the amounts of violet/blue, green/yellow, and red/NIR light transmitted by filter 119 should be comparable, so that when a gray surface illuminated by white light, as defined by light source filter 76A, is imaged by color image sensor 102, the resulting color image may be white balanced. 2) In order to effectively capture multiple reflectance images with the color image sensor 102 shown in FIGURE 5, the intensity of the reflected light received at the sensor should be approximately the same in each band of wavelengths to be detected. In the present arrangement, the relative intensity of the reflected light at the color image sensor is controlled by the design of the light source filter 76B shown in FIGURE 2.

The operation of a system based on camera 100B shown in FIGURE 5 is essentially identical to that of the first arrangement, previously described.

FIGURE 7 illustrates a third embodiment of the camera 100. Camera 100C is the same as camera 100B described above except that low light color image sensor 105 (preferably a color CCD with charge carrier multiplication such as the Texas Instruments TC252) replaces (monochrome) low light image sensor 104. In this configuration, the low light color image sensor is utilized for fluorescence imaging and the color image sensor is utilized for color imaging. The advantage of using a color low light sensor 105 in the present embodiment is that it offers the possibility for capturing images from multiple bands of wavelengths of fluorescence, which may change with pathology in different ways, as well as, capturing images from multiple bands of wavelengths of reflected light utilizing color image sensor 102 as described for the previous arrangements.

As discussed above, the filters in the light source and camera should be optimized for the imaging mode of the camera, the type of tissue to be examined and/or the type of pre-cancerous tissue to be detected, based on in vivo fluorescence and reflectance spectroscopy measurements. The preferred filter characteristics for use in the fluorescence imaging systems with a camera of the type shown in FIGURE 7 with the light source shown in FIGURE 2, operating in a fluorescence/reflectance imaging mode and a color imaging mode are shown in FIGURES 8A-8G. There are several possible configurations of such a fluorescence imaging system, operating in the fluorescence/reflectance imaging mode including i) cyan/green and red fluorescence with violet/blue and green/yellow reflectance, ii) cyan/green and red fluorescence with violet/blue and NIR reflectance, iii) cyan/green and red fluorescence with green/yellow and NIR reflectance, and iv) cyan/green and red fluorescence with violet/blue, green/yellow, and NIR reflectance. The particular configuration utilized depends on the target clinical organ and application. The filter characteristics will now be described for each of these configurations.

FIGURE 8A illustrates the composition of light transmitted by the light source filter, such as filter 76A, which is used to produce light for color imaging. This filter produces white light for use in color imaging by attenuating undesired peaks in the lamp spectrum and by correcting the color temperature of the light from the lamp.

FIGURE 8B illustrates the composition of the light transmitted by camera spectral filter 118 for the detection of fluorescence at cyan/green and red wavelengths. Used in this configuration, the filter blocks violet/blue excitation light in the range 370 - 455 nm while transmitting cyan/green light in the wavelength range of 470 - 560 nm or any desired subset of wavelengths in this range at the maximum possible transmission, and while transmitting red light in the wavelength range of 600 - 700 nm or any desired subset of wavelengths in this range at the maximum possible transmission. When used in a fluorescence and color imaging system for fluorescence/reflectance imaging, in combination with light source filter 76B described below, the filter characteristics are such that any light outside of the wavelength ranges of 470 nm - 560 nm (or any desired subset of wavelengths in this range) and 600 nm - 700 nm (or any desired subset of wavelengths in this range) contributes no more than 0.1 % to the light transmitted by the filter.

FIGURE 8C illustrates the composition of the light transmitted by light source filter 76B, which is used to produce light for fluorescence excitation and reflectance imaging at violet/blue and green/yellow wavelengths and fluorescence imaging in the cyan/green and red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image and 530 - 547 nm and/or 571 - 584 nm for oxy-hemoglobin images). Of the light transmitted by the filter, less than 0.001% is in the cyan/green fluorescence imaging wavelength range of 470 - 560 nm (or whatever desired subset of this range is specified as the transmission range of the cyan/green fluorescence wavelength band) and the red fluorescence imaging wavelength range of 600 - 700 nm (or whatever desired subset of this range is specified as the transmission range of the red fluorescence wavelength band). The light transmitted in the green/yellow wavelength range is adjusted, as part of the system design, to be an appropriate fraction of the light transmitted in the violet/blue wavelength band such that the light projected onto the color image sensor in each of these bands has comparable intensity.

FIGURE 8D illustrates the composition of light transmitted by the light source filter 76B, which is used to produce light for fluorescence excitation and reflectance imaging at NIR and green/yellow wavelengths and fluorescence imaging in the cyan/green and red, or for fluorescence excitation and reflectance imaging at NIR, green/yellow, and violet/blue wavelengths and fluorescence imaging in the cyan/green and red. This filter transmits light in the violet/blue wavelength range from 370 - 455 nm, or any desired subset of wavelengths in this range. It also transmits light in the green/yellow wavelength range of 530 - 585 nm, or any subset of wavelengths in this range (in particular 547 - 571 nm for a hemoglobin reflectance image and 530 - 547 nm and/or 571 - 584 nm for oxy-hemoglobin images). In addition, this filter transmits light in the NIR wavelength range of 700 - 900 nm, or any subset of wavelengths in this range (in particular 700 - 797 nm for a hemoglobin reflectance image and 797 - 900 nm for an oxy-hemoglobin reflectance image). Of the light transmitted by the filter, less than 0.001% is in the cyan/green fluorescence imaging wavelength range of 470 - 560 nm (or whatever desired subset of this range is specified as the transmission range of the cyan/green fluorescence wavelength band) and the red fluorescence imaging wavelength range of 600 - 700 nm (or whatever desired subset of this range is specified as the transmission range of the red fluorescence wavelength band). The light transmitted in the green/yellow wavelength range is adjusted, as part of the system design, to be an appropriate fraction of the light transmitted in the NIR wavelength band such that the light projected onto the color image sensor in each of these bands has comparable intensity.

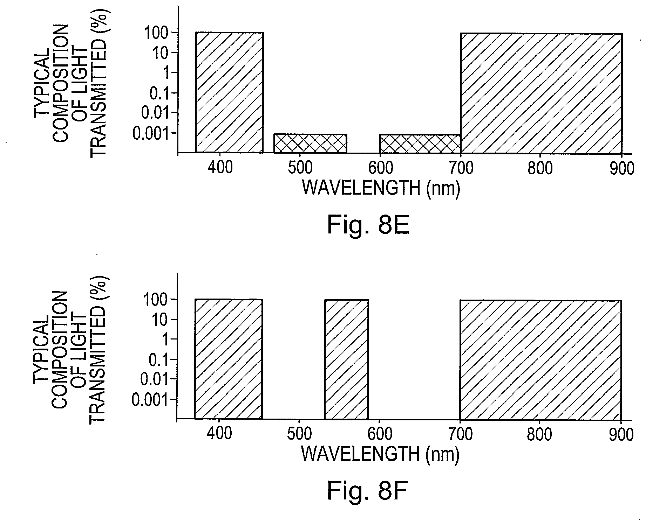

FIGURE 8E illustrates the composition of light transmitted by the light source filter 76B, which is used to produce light for fluorescence excitation and reflectance imaging at cyan/green and NIR wavelengths and fluorescence imaging in the cyan/green and red. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the NIR wavelength range of 700 - 900 nm, or any subset of wavelengths in this range (in particular 700 - 797 nm for a hemoglobin reflectance image and 797 - 900 nm for an oxy-hemoglobin reflectance image). Of the light transmitted by the filter, less than 0.001% is in the cyan/green fluorescence imaging wavelength range of 470 - 560 nm (or whatever desired subset of this range is specified as the transmission range of the cyan/green fluorescence wavelength band) and the red fluorescence imaging wavelength range of 600 - 700 nm (or whatever desired subset of this range is specified as the transmission range of the red fluorescence wavelength band). The light transmitted in the NIR wavelength range is adjusted, as part of the system design, to be an appropriate fraction of the light transmitted in the violet/blue wavelength band such that the light projected onto the color image sensor in each of these bands has comparable intensity.

FIGURE 8F illustrates the composition of the light transmitted by spectral filter 119 which is used to produce light for reflectance imaging at any combination of violet/blue, green/yellow and red/NIR wavelengths. This filter transmits light in the violet/blue wavelength range from 370 - 455 nm, or any desired subset of wavelengths in this range (in particular 390 - 423 nm for an oxy-hemoglobin reflectance image or 423 - 453 nm for a hemoglobin reflectance image). It also transmits light in the green/yellow wavelength range of 530 - 585 nm, or any subset of wavelengths in this range (in particular 547 - 571 nm for a hemoglobin reflectance image, and 530 - 547 nm and/or 571 - 584 nm for oxy-hemoglobin images). In addition, it transmits light in the red/NIR wavelength range of 700 - 900 nm, or any desired subset of wavelengths in this range (in particular 700 - 797 nm for a hemoglobin reflectance image and 797 - 900 nm for an oxy-hemoglobin reflectance image). When used in a fluorescence and color imaging system for fluorescence/reflectance imaging, in combination with light source filter 76B described above, the filter characteristics are such that any light outside of the violet/blue, green/yellow, or red/NIR wavelength ranges specified above (or any desired subset of wavelengths in those ranges) contributes no more than 0.1% to the light transmitted by the filter. The light transmitted in the red/NIR, green/yellow, and violet/blue wavelength ranges is adjusted, as part of the system design, to be such that when a gray surface illuminated by white light filtered by light source filter 76A is imaged by color image sensor 102, the resulting color image may be white balanced.

The operation of a system based on camera 100C of FIGURE 7 is similar to that of the first embodiment except that operation of the present embodiment in the fluorescence imaging mode is slightly different than that of the first embodiment due to the use of a low light color sensor 105 for the detection of fluorescence. Only the differences in operation will be explained.

The fluorescence and reflected light is transduced by low light color image sensor 105. The fluorescence and reflectance images from low light color image sensor 105 are processed, as previously described for color imaging, by processor/controller 64 to produce separate images corresponding to each of the pass bands of the mosaic filter (e.g., red, green, and blue). These separate fluorescence images, as well as the reflectance images from color image sensor 102, are encoded as video signals by processor/controller 64. A composite fluorescence/reflectance image is produced by overlaying the two fluorescence images and two (or three) reflectance images displayed in different colors on color video monitor 66. Alternatively, processor/controller 64 can produce a composite fluorescence/reflectance image by taking the difference between, or calculating the ratio of, two images, preferably one which changes with disease and one which does not change with disease or one affected by hemoglobin and one affected by oxy-hemoglobin and overlaying the resulting image, along with fluorescence and reflectance images.

The fluorescence endoscopy video systems described above have been optimized for imaging endogenous tissue fluorescence. They are not limited to this application, however, and may also be used for photo dynamic diagnosis (PDD) applications. As mentioned above, PDD applications utilize photo active drugs that preferentially accumulate in tissues suspicious for early cancer. Since effective versions of such drugs are currently in development stages, this invention does not specify the filter characteristics that are optimized for such drugs. With the appropriate light source and camera filter combinations, however, a fluorescence and color imaging system operating in fluorescence/reflectance imaging mode as described herein may be used to image the fluorescence from such drugs, as well as reflectance.

As will be appreciated, each of the embodiments of a camera for the fluorescence and color imaging system described above, due to their simplicity, naturally lend themselves to miniaturization and implementation in a fluorescence video endoscope, with the camera being incorporated into the insertion portion of the endoscope. The cameras can be utilized for both color imaging and fluorescence imaging, and in their most compact form contain no moving parts.

While the preferred embodiment of the invention has been illustrated and described, it will be appreciated that various changes can be made therein.

## Claims

1. A fluorescence imaging video system configured for acquiring color and multichannel fluorescence/reflectance images, including:
an insertion portion of an endoscope; a single multi-mode light source (52) adapted to produce both multi-wavelength excitation light for fluorescence imaging and illumination light having red, green and blue components,
a plurality of light source filters (76A, 76B) positionable between the light source (52) and an illumination optical transmission system (54), adapted to transmit substantially all the multi-wavelength excitation light intensity and adapted to transmit selectively a predetermined portion of one or more of the red, green and blue component intensity;
the optical transmission system (54) being adapted to direct the filtered light to a tissue sample (58) and an imaging optical transmission system (62) adapted to collect reflected light and multi-wavelength fluorescence light produced by the tissue;
a camera (100C), located in said insertion portion, and adapted to receive the light collected by the optical transmission system (62), the camera including:
a first color image sensor (102), and
a second color image sensor (105) having spectral filter (118), wherein each of the light source filters (76A, 76B) are adapted to be positioned stationarily during an imaging mode,
the camera (100C) further comprises a beam splitter that is adapted to direct reflectance images onto the first color image sensor (102) and multi-wavelength and fluorescence light onto the second color image sensor (105),
the first color image sensor (102) has a first spectral filter (119) positioned in front of the color image sensor (102) said filter being adapted to block selectively the multi-wavelength excitation light and transmit reflectance light at wavelengths other than the multi-wavelength excitation light, and
the spectral filter (118) of the second color image sensor (105) is a second spectral filter (118), positioned in front of the color image sensor (105) said filter being adapted to block selectively the multi-wavelength excitation light and transmit multi-wavelength fluorescence light at wavelengths other than the multi-wavelength excitation light.

2. The system of claim 1, further comprising an image processor/controller (64) adapted to receive image signals from the first (102) and second (105) color image sensors and is adapted to form video signals representing color and/or multi-wavelength fluorescence/reflectance images.

3. The system of claim 1 or claim 2, further comprising a color video monitor (66) for displaying a white-balanced color image and/or a multi-wavelength fluorescence/reflectance image from the image signals.

4. The system of one of the preceding claims, wherein a light source filter (76B) of the multi-mode light source (52) is adapted to transmit the multi-wavelength excitation light and an amount of reference reflectance light not in a multi-wavelength fluorescence detection wavelength band and adapted substantially to block transmission of light from the multi-mode light source (52) at wavelengths in the multi-wavelength fluorescence detection wavelength band.

5. The system of one of the preceding claims, wherein for detection of fluorescence at cyan/green and red wavelengths the second filter (118) is adapted to block violet/blue excitation light in the range 370-455 nm while transmitting cyan/green fluorescence light in the wavelength range of 470 -560 nm or any desired subset of wavelengths in this range, and while transmitting red light in the wavelength range of 600-700 nm or any desired subset of wavelengths in this range.

6. The system of claim 4 or 5, wherein for fluorescence excitation and reflectance imaging at violet/blue and green/yellow wavelengths and fluorescence imaging in the cyan/green and red, the light source filter (76B) is adapted to transmit light in the violet/blue wavelength range from 370-455 nm, or any desired subset of wavelengths in this range, in particular 390-423 nm for oxy-hemoglobin reflectance imaging or 423-453 nm for a hemoglobin reflectance imaging, and also transmit light in the green/yellow wavelength range of 530-585 nm, or any subset of wavelengths in this range, in particular 547-571 nm for hemoglobin reflectance imaging and 530-547 nm and/or 571-584 nm for oxy-hemoglobin imaging, while substantially blocking light transmission in the cyan/green fluorescence imaging wavelength range of 470-560 nm and the red fluorescence imaging wavelength range of 600-700 nm.

7. The system of one of claims 4-6, wherein the ratio of the light transmitted by the light source filter (76B) in the green/yellow wavelength range to the light transmitted in the violet/blue wavelength range is adjusted, such that the combined light projected onto the first color image sensor (102) in each of these ranges has comparable intensity.

8. The system of one of claims 4-6, wherein for fluorescence imaging in the cyan/green and red, and fluorescence excitation and reflectance imaging at NIR and/or green/yellow and/or violet/blue wavelengths, the light source filter (76B) is adapted to transmit light in the violet/blue wavelength range from 370-455 nm, or any desired subset of wavelengths in this range, and also transmit light in the green/yellow wavelength range of 530-585 nm, or any subset of wavelengths in this range, in particular 547-571 nm for hemoglobin reflectance imaging and 530-547 nm and/or 571-584 nm for oxyhemoglobin imaging, and also transmit light in the NIR wavelength range of 700-900 nm, or any subset of wavelengths in this range, in particular 700-797 nm for hemoglobin reflectance imaging and 797-900 nm for oxyhemoglobin reflectance imaging.

9. The system of comprising a color video monitor (66), wherein the image processor/controller (64) is adapted to produce a composite fluorescence/reflectance image comprising an image created from green fluorescence light and an image created from red reflectance light and adapted to superimpose and display said images in different colors on the color video monitor (66).

## Patentansprüche

1. Fluoreszenz-Bildgebungsvideosystem, konfiguriert zum Erfassen von Farb- und Mehrkanal-Fluoreszenz-/Reflektanzbildern, umfassend:
einen Einführungsabschnitt eines Endoskops; eine einzelne Multimodus-Lichtquelle (52), geeignet zum Erzeugen von sowohl Erregungslicht mit mehreren Wellenlängen für die Fluoreszenz-Bildgebung als auch Beleuchtungslicht mit roten, grünen und blauen Komponenten,
mehrere Lichtquellenfilter (76A, 76B), positionierbar zwischen der Lichtquelle (52) und einem optischen Beleuchtungsübertragungssystem (54), geeignet zur Übertragung von im Wesentlichen der gesamten Intensität des Beleuchtungslichts mit mehreren Wellenlängen und geeignet zur selektiven Übertragung eines vorbestimmten Teils der Intensität von einer oder mehr der roten, grünen und blauen Komponenten;
welches optische Übertragungssystem (54) geeignet ist, um das gefilterte Licht auf eine Gewebeprobe (58) und ein optisches Bildgebungsübertragungssystem (62), geeignet zum Sammeln von reflektiertem Licht und Fluoreszenzlicht mit mehreren Wellenlängen, erzeugt von dem Gewebe, zu richten;
eine Kamera (100C), angeordnet in dem Einführungsabschnitt und geeignet zum Aufnehmen des Lichts, gesammelt von dem optischen Übertragungssystem (62), welche Kamera Folgendes umfasst:
einen ersten Farbbildsensor (102) und
einen zweiten Farbbildsensor (105) mit Spektralfilter (118),
wobei jeder der Lichtquellenfilter (76A, 76B) geeignet ist, um ortsfest während eines Bildgebungsmodus positioniert zu werden,
die Kamera (100C) ferner einen Strahlenteiler umfasst, der geeignet ist, Reflektanzbilder auf den ersten Farbbildsensor (102) und Fluoreszenzlicht mit mehreren Wellenlängen auf den zweiten Farbbildsensor (105) zu richten,
der erste Farbbildsensor (102) einen ersten Spektralfilter (119) hat, der vor dem Farbbildsensor (102) positioniert ist, welcher Filter geeignet ist, um das Erregungslicht mit mehreren Wellenlängen selektiv zu blockieren und das Reflektanzlicht mit Wellenlängen außer dem Erregungslicht mit mehreren Wellenlängen zu übertragen, und
der Spektralfilter (118) des zweiten Farbbildsensors (105) ein zweiter Spektralfilter (118) ist, der vor dem Farbbildsensor (105) positioniert ist, welcher Filter geeignet ist, um das Erregungslicht mit mehreren Wellenlängen selektiv zu blockieren und das Fluoreszenzlicht mit Wellenlängen außer dem Erregungslicht mit mehreren Wellenlängen zu übertragen.

2. System nach Anspruch 1, ferner umfassend einen Bildprozessor/- controller (64), geeignet zum Empfangen von Bildsignalen von den ersten (102) und zweiten (105) Farbbildsensoren und geeignet zum Bilden von Videosignalen, die Fluoreszenz-/Reflektanzbilder in Farbe und/oder mit mehreren Wellenlängen darstellen.

3. System nach Anspruch 1 oder Anspruch 2, ferner umfassend einen Farbvideomonitor (66) zum Anzeigen eines weißabgeglichenen Farbbildes und/oder eines Fluoreszenz-/Reflektanzbildes mit mehreren Wellenlängen anhand der Bildsignale.

4. System nach einem der vorhergehenden Ansprüche, wobei ein Lichtquellenfilter (76B) der Multimodus-Lichtquelle (52) geeignet ist, um das Erregungslicht mit mehreren Wellenlängen und eine Menge von Referenzreflektanzlicht nicht in einem Fluoreszenzdetektions-Wellenlängenband mit mehreren Wellenlängen zu übertragen, und geeignet ist, um im Wesentlichen die Übertragung von Licht von der Multimodus-Lichtquelle (52) bei Wellenlängen in dem Fluoreszenzdetektions-Wellenlängenband mit mehreren Wellenlängen zu blockieren.

5. System nach einem der vorhergehenden Ansprüche, wobei für die Detektion von Fluoreszenz bei cyanfarbenen/grünen und roten Wellenlängen der zweite Filter (118) geeignet ist, um violettes/blaues Erregungslicht im Bereich von 370-455 nm zu blockieren, während cyanfarbenes/grünes Fluoreszenzlicht im Wellenlängenbereich von 470-560 nm oder einem beliebigen gewünschten Teilbereich von Wellenlängen in diesem Bereich übertragen wird und während rotes Licht im Wellenbereich von 600-700 nm oder einem beliebigen Teilbereich von Wellenlängen in diesem Bereich übertragen wird.

6. System nach Anspruch 4 oder 5, wobei für die Fluoreszenz-Bildgebung und Reflektanz-Bildgebung bei violetten/blauen und grünen/gelben Wellenlängen und Fluoreszenz-Bildgebung in den cyanfarbenen/grünen und roten Wellenlängen der Lichtquellenfilter (76B) geeignet ist, das Licht in dem violetten/blauen Wellenlängenbereich von 370-455 nm oder einem beliebigen gewünschten Teilbereich von Wellenlängen in diesem Bereich, insbesondere 390-423 nm für Oxyhämoglobin-Reflektanz-Bildgebung oder 423-453 nm für eine Hämoglobin-Reflektanz-Bildgebung zu übertragen und auch Licht im grünen/gelben Wellenlängenbereich von 530-585 nm oder jedem beliebigen Teilbereich von Wellenlängen in diesem Bereich, insbesondere 547-571 nm für Hämoglobin-Reflektanz-Bildgebung und 530-547 nm und/oder 571-584 nm für Oxyhämoglobin-Bildgebung zu übertragen, während im Wesentlichen die Lichtübertragung in dem cyanfarbenen/grünen Fluoreszenz-Bildgebungs-Wellenlängenbereich von 470-560 nm und dem roten Fluoreszenz-Bildgebungs-Wellenbereich von 600-700 nm blockiert wird.

7. System nach einem der Ansprüche 4-6, wobei das Verhältnis des Lichts, das von dem Lichtquellenfilter (76B) in dem grünen/gelben Wellenlängenbereich übertragen wird, zu dem Licht, das in dem violetten/blauen Wellenlängenbereich übertragen wird, so angepasst ist, dass das kombinierte Licht, das auf den ersten Farbbildsensor (102) in jedem dieser Bereiche projiziert wird, eine vergleichbare Intensität hat.

8. System nach einem der Ansprüche 4-6, wobei für die Fluoreszenz-Bildgebung im cyan/grünen und roten Wellenlängenbereich und die Fluoreszenzerregungs- und Reflektanz-Bildgebung bei NIR und/oder grünen/gelben und/oder violetten/blauen Wellenlängen der Lichtquellenfilter (76B) geeignet ist, um Licht im violetten/blauen Wellenlängenbereich von 370-455 nm oder jedem beliebigen gewünschten Teilbereich von Wellenlängen in diesem Bereich zu übertragen und auch Licht im grünen/gelben Wellenlängenbereich von 530-585 nm oder einem beliebigen Teilbereich von Wellenlängen in diesem Bereich, insbesondere 547-571 nm für Hämoglobin-Reflektanz-Bildgebung und 530-547 nm und/oder 571-584 nm für Oxyhämoglobin-Bildgebung, zu übertragen und auch Licht im NIR-Wellenlängenbereich von 700-900 nm oder einem beliebigen Teilbereich von Wellenlängen in diesem Bereich, insbesondere 700-797 nm für Hämoglobin-Reflektanz-Bildgebung und 797-900 nm für Oxyhämoglobin-Reflektanz-Bildgebung, zu übertragen.

9. System nach Anspruch 6, umfassend einen Farbvideomonitor (66), wobei der Bildprozessor/-controller (64) geeignet ist, um ein zusammengesetztes Fluoreszenz-/Reflektanzbild zu erzeugen, das ein Bild, erstellt von grünem Fluoreszenzlicht, und ein Bild, erstellt von rotem Reflektanzlicht, umfasst, und geeignet ist, um die Bilder in verschiedenen Farben auf dem Farbvideomonitor (66) übereinanderzulegen und anzuzeigen.

## Revendications

1. Système vidéo d'imagerie par fluorescence configuré pour acquérir des images par fluorescence/par réflectance multicanaux, comprenant :
une partie d'insertion d'un endoscope ; une source de lumière multi-mode (52) adaptée pour produire une lumière d'excitation multi-longueurs d'onde pour l'imagerie par fluorescence et une lumière d'éclairage ayant des composantes rouges, vertes et bleues,
une pluralité de filtres de source de lumière (76A, 76B) positionnables entre la source de lumière (52) et un système de transmission optique d'éclairage (54), adaptés pour transmettre sensiblement l'intégralité de l'intensité de la lumière d'excitation multi-longueurs d'onde et adaptés pour transmettre sélectivement une partie prédéterminée de l'intensité d'une ou plusieurs des composantes rouges, vertes et bleues ;
le système de transmission optique (54) étant adapté pour orienter la lumière filtrée vers un échantillon de tissu (58) et un système de transmission optique d'imagerie (62) adapté pour collecter la lumière réfléchie et la lumière de fluorescence multi-longueurs d'onde produite par le tissu ;
un appareil photo (100C), situé dans ladite partie d'insertion, et adapté pour recevoir la lumière collectée par le système de transmission optique (62), l'appareil photo comprenant :
un premier capteur d'image en couleur (102), et
un second capteur d'image en couleur (105) ayant un filtre spectral (118),
dans lequel chacun des filtres de source de lumière (76A, 76B) est adapté pour être positionné de manière stationnaire pendant un mode d'imagerie,
l'appareil photo (100C) comprend en outre un séparateur de faisceau qui est adapté pour orienter les images par réflectance vers le premier capteur d'image en couleur (102) et la lumière à multi-longueurs d'onde et de fluorescence vers le second capteur d'image en couleur (105),
le premier capteur d'image en couleur (102) possède un premier filtre spectral (119) positionné en face du capteur d'image en couleur (102), ledit filtre étant adapté pour bloquer sélectivement la lumière d'excitation multi-longueurs d'onde et pour transmettre la lumière de réflectance à des longueurs d'onde différentes de la lumière d'excitation multi-longueurs d'onde, et
le filtre spectral (118) du second capteur d'image en couleur (105) est un second filtre spectral (118), positionné en face du capteur d'image en couleur (105), ledit filtre étant adapté pour bloquer sélectivement la lumière d'excitation multi-longueurs d'onde et pour transmettre la lumière de fluorescence multi-longueurs d'onde à des longueurs d'onde différentes de la lumière d'excitation multi-longueurs d'onde.

2. Système selon la revendication 1, comprenant en outre un processeur/contrôleur d'images (64) adapté pour recevoir des signaux d'images de la part du premier (102) et du second (105) capteurs d'images en couleur, et adapté pour former des signaux vidéo représentant des images par fluorescence/réflectance en couleur et/ou multi-longueurs d'onde.

3. Système selon la revendication 1 ou 2, comprenant en outre un moniteur vidéo couleur (66) destiné à afficher une image en couleur à équilibrage des blancs et/ou une image par fluorescence/réflectance multi-longueurs d'onde à partir des signaux d'images.

4. Système selon l'une des revendications précédentes, dans lequel un filtre de source de lumière (76B) de la source de lumière multimodes (52) est adapté pour transmettre la lumière d'excitation multi-longueurs d'onde et une quantité de lumière de réflectance de référence qui ne se trouve pas sur une bande de longueurs d'onde de détection de fluorescence multi-longueurs d'onde, et adapté pour bloquer sensiblement la transmission de lumière depuis la source de lumière multimodes (52) aux longueurs d'onde de la bande de longueurs d'onde de détection de fluorescence multi-longueurs d'onde.

5. Système selon l'une des revendications précédentes, dans lequel, pour la détection de fluorescence aux longueurs d'onde cyan/vertes et rouges, le second filtre (118) est adapté pour bloquer la lumière d'excitation violette/bleue sur la plage de 370 à 455 nm tout en transmettant la lumière de fluorescence cyan/verte sur la plage de longueurs d'onde de 470 à 560 nm, ou n'importe quel sous-ensemble de longueurs d'onde sur cette plage, tout en transmettant la lumière rouge sur la plage de longueurs d'onde de 600 à 700 nm, ou n'importe quel sous-ensemble de longueurs d'onde sur cette plage.

6. Système selon la revendication 4 ou 5, dans lequel, pour l'excitation par fluorescence et l'imagerie par réflectance aux longueurs d'onde violettes/bleues et vertes/jaunes, et l'imagerie par fluorescence aux longueurs d'onde cyan/vertes et rouges, le filtre de source de lumière (76B) est adapté pour transmettre la lumière sur la plage de longueurs d'onde violettes/bleues de 370 à 455 nm, ou n'importe quel sous-ensemble de longueurs d'onde sur cette plage, en particulier de 390 à 423 nm pour l'imagerie par réflectance de l'oxyhémoglobine, ou de 423 à 453 nm pour une imagerie par réflectance de l'hémoglobine, et pour transmettre la lumière sur la plage de longueurs d'onde vertes/jaunes de 530 à 585 nm, ou n'importe quel sous-ensemble de longueurs d'onde sur cette plage, en particulier de 547 à 571 nm pour l'imagerie par réflectance de l'hémoglobine, et de 530 à 547 nm et/ou de 571 à 584 nm pour l'imagerie de l'oxyhémoglobine, tout en bloquant sensiblement la transmission de lumière sur la plage de longueurs d'onde cyan/vertes d'imagerie par fluorescence de 470 à 560 nm, et sur la plage de longueurs d'onde rouges d'imagerie par fluorescence de 600 à 700 nm.

7. Système selon l'une des revendications 4 à 6, dans lequel le rapport entre la lumière transmise par le filtre de source de lumière (76B) sur la plage de longueurs d'onde vertes/jaunes et la lumière transmise sur la plage de longueurs d'onde violettes/bleues est ajusté, de sorte que la lumière combinée projetée vers le premier détecteur d'image en couleur (102) sur chacune de ces plages possède une intensité comparable.

8. Système selon l'une des revendications 4 à 6, dans lequel, pour l'imagerie par fluorescence sur la plage de longueurs d'onde cyan/vertes et rouges, et pour l'imagerie par excitation de fluorescence et par réflectance sur la plage de longueurs d'onde NIR et/ou vertes/jaunes et/ou violettes/bleues, le filtre de source de lumière (76B) est adapté pour transmettre la lumière sur la plage de longueurs d'onde violettes/bleues de 370 à 455 nm, ou n'importe quel sous-ensemble souhaité de longueurs d'onde sur cette plage, et pour transmettre la lumière sur la plage de longueurs d'onde vertes/jaunes de 530 à 585 nm, ou n'importe quel sous-ensemble de longueurs d'onde sur cette plage, en particulier de 547 à 571 nm pour l'imagerie par réflectance de l'hémoglobine, et de 530 à 547 nm et/ou 571 à 584 nm pour l'imagerie de l'oxyhémoglobine, et pour transmettre la lumière sur la plage de longueurs d'onde NIR de 700 à 900 nm, ou n'importe quel sous-ensemble de longueurs d'onde sur cette plage, en particulier de 700 à 797 nm pour l'imagerie par réflectance de l'hémoglobine, et de 797 à 900 nm pour l'imagerie par réflectance de l'oxyhémoglobine.

9. Système comprenant un moniteur vidéo couleur (66), dans lequel le processeur/contrôleur d'images (64) est adapté pour produire une image par fluorescence/réflectance composite comprenant une image créée à partir d'une lumière de fluorescence verte et une image créée à partir d'une lumière de réflectance rouge, et adapté pour superposer et afficher lesdites images en différentes couleurs sur le moniteur vidéo couleur (66).
